# EUROPEAN PATENT APPLICATION

(11) **EP 4 691 343 A1**
(43) Date of publication of application: **11.02.2026**
(21) Application number: 24779106.4
(22) Date of filing: 04.03.2024
(51) Int. Cl.: A61B 3/10

(54) **TEAR FILM IMAGING DEVICE**

(30) Priority: 24.03.2023 JP 2023048324
(71) Applicant: KOWA COMPANY, LTD., Naka-ku, Nagoya-shi, Aichi 460-8625 (JP)
(72) Inventor: MATSUMURA, Kazunori, Chofu-shi Tokyo 182-0021 (JP); KAWAI, Yoshiharu, Chofu-shi Tokyo 182-0021 (JP)
(74) Representative: Mathys & Squire
(86) International application number: PCT/JP2024/007999
(87) International publication number: WO 2024/202968

(57) **Abstract**

In order to provide a tear film photographing device capable of implementing a position detection function for an eye under examination and an automatic alignment function, the tear film photographing device includes: a first optical system that includes an objective lens that is disposed so as to face the eye under examination, a first illumination unit that irradiates the eye under examination with first light through the objective lens, and a first photographing unit that receives the first light reflected by a tear film of the eye under examination and passing through the objective lens and photographs interference fringes formed on the tear film; a second optical system that includes a second illumination unit that irradiates the eye under examination with second light, a second light reflection unit that is disposed on an optical path of the first optical system and selectively reflects the second light reflected by a corneal surface of the eye under examination and passing through the objective lens, and a second photographing unit that receives the second light through the second light reflection unit and photographs an anterior eye portion of the eye under examination, the second optical system adjusting a positional relationship between respective optical components such that the second photographing unit can photograph a wider range than that of the first photographing unit; and a positional deviation amount calculation unit that calculates a positional deviation amount from a proper position of the first optical system with respect to the eye under examination based on an image of the anterior eye portion obtained by the second optical system.

## Description

### Technical Field

The present invention relates to a tear film photographing device for photographing a tear film of an eye under examination.

### Background Art

In recent years, the number of dry eye patients has been increasing due to aging or an increase in work using digital devices such as personal computers, and appropriate examinations and treatments for dry eye have been required. Observation of a tear film of an eye under examination is essential for the dry eye examination, and Patent Literature 1 has been proposed as a device for observing the tear film, for example.

In addition, since it is required to clearly capture the tear film when observing the tear film, appropriate alignment of the device with the eye under examination becomes a challenge. For example, Patent Literature 2 has been proposed as a device for solving such an alignment problem. A tear film photographing device described in Patent Literature 2 has a positional deviation detection function and an automatic alignment function of automatically moving a tear film photographing optical system according to a detected positional deviation amount.

### Citation List

### Patent Literature

Patent Literature 1: JP H10-33483 A
Patent Literature 2: WO 2021/066124 A

### Summary of Invention

### Technical Problem

In the tear film photographing device described in Patent Literature 2, it is necessary to arrange a detector and a light emitting diode (LED) on a back side of an objective lens when viewed from the eye under examination, and there is a problem that it is difficult to implement such a device in a case where there is a space limitation inside the optical system. Specifically, in the case of attempting to design with a specification in which an image magnification changes when a distance between the eye and the objective lens changes, there is a problem that an effective diameter required for the lens after branching by a mirror is considerably large, and it is thus difficult to neatly arrange three optical systems in parallel. In addition, in a case where optical paths of the LED and the detector are folded on the back side of the objective lens, influences of positional variations of the objective lens and a folding mirror and an angular variation of the folding mirror are added, and thus adjustment of positions of the LED and the detector may become more difficult, which is problematic.

The present invention has been made in view of the above problems, and an object of the present invention is to provide a tear film photographing device capable of implementing a position detection function for an eye under examination and an automatic alignment function without an increase in size of the entire device.

### Solution to Problem

A tear film photographing device according to the present invention is a tear film photographing device that photographs interference fringes formed on a tear film of an eye under examination, the tear film photographing device including: a first optical system that includes an objective lens that is disposed so as to face the eye under examination, a first illumination unit that irradiates the eye under examination with first light through the objective lens, and a first photographing unit that receives the first light reflected by the tear film of the eye under examination and passing through the objective lens and photographs the interference fringes formed on the tear film; a second optical system that includes a second illumination unit that irradiates the eye under examination with second light, a second light reflection unit that is disposed on an optical path of the first optical system and selectively reflects the second light reflected by a corneal surface of the eye under examination and passing through the objective lens, and a second photographing unit that receives the second light through the second light reflection unit and photographs an anterior eye portion of the eye under examination, the second optical system adjusting a positional relationship between respective optical components such that a photographing range in the second photographing unit is wider than a photographing range in the first photographing unit; and a positional deviation amount calculation unit that calculates a positional deviation amount from a proper position of the first optical system with respect to the eye under examination based on an image of the anterior eye portion obtained by the second optical system.

Furthermore, in the tear film photographing device according to the present invention, the second illumination unit may include a plurality of first positional deviation detection light sources that are disposed around the objective lens and emit the second light, the second optical system may capture the image of the anterior eye portion and photograph a plurality of bright spots of a Purkinje image of the second light, and the positional deviation amount calculation unit may calculate the positional deviation amounts in an X direction and a Y direction based on the image of the anterior eye portion and the plurality of bright spots.

Furthermore, in the tear film photographing device according to the present invention, the positional deviation amount calculation unit may have a first positional deviation amount calculation function of detecting the center of a pupil of the eye under examination and calculating the positional deviation amounts in the X direction and the Y direction based on the image of the anterior eye portion obtained by the second optical system.

Furthermore, in the tear film photographing device according to the present invention, the positional deviation amount calculation unit may have a second positional deviation amount calculation function of detecting the center of the plurality of bright spots and calculating the positional deviation amounts in the X direction and the Y direction based on images of the plurality of bright spots obtained by the second optical system.

Furthermore, in the tear film photographing device according to the present invention, the positional deviation amount calculation unit may have a third positional deviation amount calculation function of calculating a distance between the images of the respective bright spots among the images of the plurality of bright spots obtained by the second optical system and calculating the positional deviation amount in a Z direction based on the distance.

Furthermore, the tear film photographing device according to the present invention may further include a Z-direction detection unit that includes a second positional deviation detection light source that is disposed around the objective lens and irradiates the eye under examination with third light and a light receiving sensor that is disposed around the objective lens and receives the third light reflected by the anterior eye portion of the eye under examination, in which the positional deviation amount calculation unit may have a fourth positional deviation amount calculation function of detecting the positional deviation amount in a Z direction based on a light receiving position for the third light in the light receiving sensor.

Furthermore, in the tear film photographing device according to the present invention, the plurality of first positional deviation detection light sources may include three first positional deviation detection light sources disposed at positions of respective vertexes of an inverted isosceles triangle shape when viewed from a front side of the objective lens.

### Advantageous Effects of Invention

One or more deficiencies are solved by the embodiments of the present application.

### Brief Description of Drawings

Fig. 1 is a system block diagram illustrating an example of a configuration of a tear film photographing device 1 corresponding to an embodiment of the present invention.
Fig. 2 is a schematic diagram illustrating an example of a configuration of an optical system unit 10 in the tear film photographing device 1 corresponding to the embodiment of the present invention.
Fig. 3 is an optical path diagram for describing an optical path of a first optical system and an optical path of a second optical system in the tear film photographing device 1 corresponding to the embodiment of the present invention.
Fig. 4 is an explanatory diagram illustrating an outline of position correction processing in the tear film photographing device 1 corresponding to the embodiment of the present invention.
Fig. 5 is a flowchart illustrating a flow of the position correction processing in the tear film photographing device 1 corresponding to the embodiment of the present invention.
Fig. 6 is an explanatory diagram illustrating an example of installation of a second illumination unit 121 in the tear film photographing device 1 corresponding to the embodiment of the present invention and a method of using a bright spot.
Fig. 7 is an explanatory diagram illustrating a method of calculating a positional deviation amount in a Z direction using a signal from a Z-direction detection unit 13 in the tear film photographing device 1 corresponding to the embodiment of the present invention.

### Description of Embodiments

Hereinafter, an example of a tear film photographing device according to an embodiment of the present invention will be described with reference to the drawings. The tear film photographing device according to the present example is a device for photographing a tear film of an eye of a subject (hereinafter, referred to as "eye under examination").

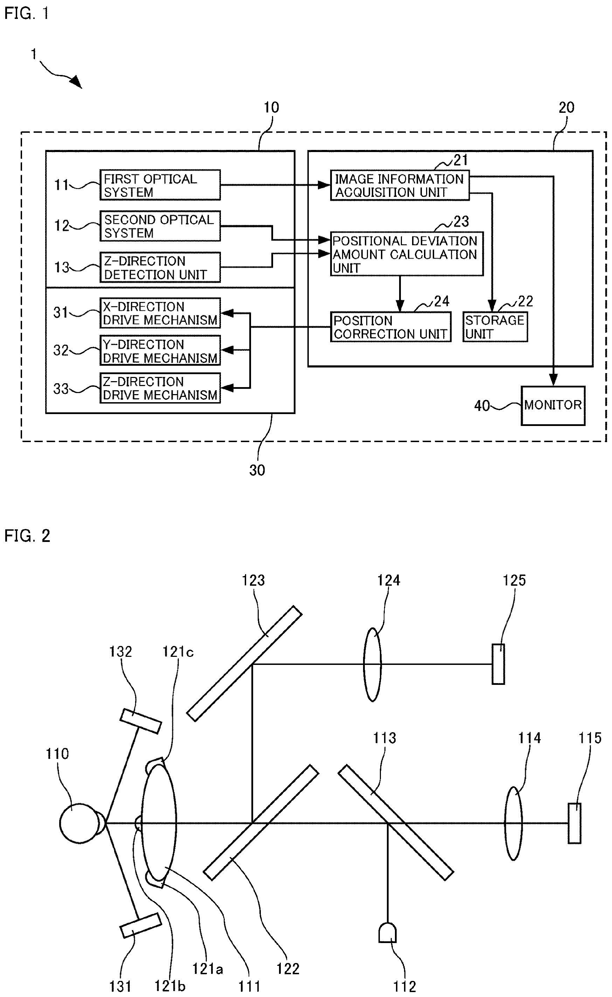

Fig. 1 is a system block diagram illustrating an example of a configuration of a tear film photographing device 1 corresponding to the embodiment of the present invention. As illustrated in Fig. 1, the tear film photographing device 1 includes an optical system unit 10, a control unit 20 that executes various types of arithmetic processing for implementing positional deviation detection and automatic alignment based on various types of information output from the optical system unit 10, a drive mechanism unit 30 that moves a stage supporting the optical system unit 10 based on a result of the arithmetic processing executed by the control unit 20, and a monitor 40 that displays a photographing target photographed through the optical system unit 10.

The optical system unit 10 includes a first optical system 11, a second optical system 12, and a Z-direction detection unit 13. In the present embodiment, optical members belonging to the optical systems are, for example, positioned on an upper side of the tear film photographing device 1 and accommodated in an optical member accommodation casing (not illustrated) attached at a position facing the eye under examination. In addition, it is preferable that a stage (not illustrated) supporting the optical members belonging to the first optical system 11, the second optical system 12, and the Z-direction detection unit 13 is accommodated in the optical member accommodation casing.

The control unit 20 includes an image information acquisition unit 21 and a storage unit 22. The image information acquisition unit 21 acquires captured image data of the tear film output from the first optical system 11. In addition, the storage unit 22 stores the captured image data acquired by the image information acquisition unit 21. Furthermore, the captured image data acquired by the image information acquisition unit 21 may be displayed on the monitor 40.

Furthermore, the control unit 20 includes a positional deviation amount calculation unit 23 and a position correction unit 24. The positional deviation amount calculation unit 23 has a function of calculating a positional deviation amount between the tear film photographing device 1 and the eye under examination based on a position detection image output from the second optical system 12 and triangulation information output from the Z-direction detection unit 13. In addition, the positional deviation amount calculation unit 23 transmits information regarding the positional deviation amount to the position correction unit 24. The position correction unit 24 calculates position correction information based on the received information regarding the positional deviation amount and transmits the position correction information to the drive mechanism unit 30.

The control unit 20 may be a general computer including a central processing unit (CPU), a random access memory (RAM), a hard disk drive, an input/output device, a communication interface, and the like. The respective functions executed by the image information acquisition unit 21, the storage unit 22, the positional deviation amount calculation unit 23, and the position correction unit 24 described above may be implemented by the CPU or the like that executes desired arithmetic processing according to a program stored in advance. Furthermore, the control unit 20 may be configured to be able to communicate with an external information terminal via the communication interface.

The drive mechanism unit 30 includes an X-direction drive mechanism 31 that moves the stage supporting the optical members belonging to the first optical system 11, the second optical system 12, and the Z-direction detection unit 13 in an X direction, a Y-direction drive mechanism 32 that moves the stage in a Y direction, and a Z-direction drive mechanism 33 that moves the stage in a Z direction. Configurations of the X-direction drive mechanism 31, the Y-direction drive mechanism 32, and the Z-direction drive mechanism 33 are not particularly limited as long as the stage can be moved in the corresponding directions. For example, each drive mechanism may include a motor, a gear connected to a shaft portion of the motor, and a rail member meshing with the gear. In this case, the motor receives the position correction information from the position correction unit 24 and rotates the shaft at a rotation speed corresponding to the received position correction information. The rail member is a member provided at a predetermined position on the stage and converts a rotational motion of the shaft of the motor transmitted via the gear into a translational motion in the X, Y, and Z directions.

Fig. 2 is a schematic diagram illustrating an example of a configuration of the optical system unit 10 in the tear film photographing device 1 corresponding to the embodiment of the present invention. In Fig. 2, the first optical system 11 includes an objective lens 111 facing an eye 110 under examination including the tear film, a first illumination unit 112 for irradiating the eye 110 under examination with first light through the objective lens, a half mirror 113, an imaging lens 114, and a first photographing unit 115.

The first illumination unit 112 is not particularly limited as long as the first illumination unit 112 can illuminate the tear film of the eye 110 under examination. However, the first illumination unit 112 is preferably a white light source in order to allow an image of the tear film to be accurately visually recognized through the monitor 40. Examples of the white light source include an incandescent lamp, a halogen lamp, a white light emitting diode (LED) lamp, and a fluorescent lamp.

The first photographing unit 115 is configured to receive the first light reflected by the tear film of the eye 110 under examination and passing through the objective lens 111 and photograph interference fringes formed on the tear film. The first photographing unit 115 is not particularly limited as long as the first photographing unit 115 can capture the image of the tear film illuminated by the first illumination unit 112. Examples of the first photographing unit 115 include a charge-coupled device (CCD) image sensor and a complementary metal-oxide-semiconductor (CMOS) image sensor. The captured image data of the tear film obtained by the first photographing unit 115 is transmitted to the control unit 20.

In the first optical system 11, illumination light from the first illumination unit 112 travels along the following optical path. That is, the illumination light (first light) emitted from the first illumination unit 112 reaches the half mirror 113, and a part thereof is reflected toward the objective lens 111. The reflected illumination light passes through the objective lens 111, then reaches the eye under examination, is specularly reflected at the tear film of the eye under examination, and is emitted in a direction opposite to the incoming optical path. The specularly reflected illumination light passes through the objective lens 111 again and then passes through the half mirror 113. Further, the illumination light passing through the half mirror 113 passes through the imaging lens 114 and is formed as the image by the first photographing unit 115. As a result, the image of the tear film is captured.

Next, in Fig. 2, the second optical system 12 includes a second illumination unit 121 that irradiates the eye under examination with second light, a second light reflection unit 122 disposed on the same optical path as the first optical system 11, a mirror 123, an imaging lens 124, and a second photographing unit 125.

As illustrated in Fig. 2, the second illumination unit 121 may be a plurality of first positional deviation detection light sources 121a to 121c that are disposed around the objective lens 11 and emit the second light. Various light sources can be adopted as the first positional deviation detection light sources 121a to 121c as long as the light sources can emit the second light used for detecting a position of the optical system unit 10 with respect to the eye 110 under examination. However, the first positional deviation detection light sources 121a to 121c are preferably light sources capable of separating the second light from the first light while sharing a common optical axis, and for example, it is conceivable to adopt near-infrared LEDs as light sources having different wavelengths. In addition, it is preferable to adopt three first positional deviation detection light sources 121a to 121c so that position detection can be performed by a method to be described later. A state in which bright spots irradiated by the three first positional deviation detection light sources 121a to 121c are reflected on an anterior eye portion of the eye 110 under examination is photographed, and the positional deviation can be detected using information regarding the three bright spots appearing in the captured image.

As the second light reflection unit 122, a member that is disposed on the same optical path as the first optical system 11 and selectively reflects the second light reflected by a corneal surface of the eye under examination and passing through the objective lens is adopted. Any member may be adopted as long as the second light can be selectively reflected. For example, it is conceivable to adopt a dichroic mirror having a property of reflecting light having a specific wavelength and transmitting light having other wavelengths.

The second photographing unit 125 receives the second light via the second light reflection unit 122 and photographs the anterior eye portion of the eye 110 under examination. The second photographing unit 125 is not particularly limited as long as the second photographing unit 125 can capture an image of the anterior eye portion of the eye under examination illuminated by the second illumination unit 121. Examples of the second photographing unit 125 include a CCD image sensor and a CMOS image sensor. Captured image data of the anterior eye portion of the eye under examination obtained by the second photographing unit 125 is transmitted to the control unit 20.

In the second optical system 12, illumination light from the second illumination unit 121 (first positional deviation detection light sources 121a to 121c) travels along the following optical path. That is, the second light emitted from the second illumination unit 121 is specularly reflected by the anterior eye portion of the eye under examination and is incident on the objective lens 111. The specularly reflected second light passes through the objective lens 111 and then reaches the second light reflection unit 122. The second light is selectively reflected by the second light reflection unit 122, passes through the imaging lens 124 while being reflected by the mirror 123, and is formed as the image by the second photographing unit 125. As a result, the image of the anterior eye portion of the eye under examination irradiated with the second light is captured.

Here, a positional relationship between optical components is adjusted such that a photographing range in the second photographing unit 125 is wider than a photographing range in the first photographing unit 115. The captured image in the second photographing unit 125 is desired to be used for positional deviation detection, and thus, a state in which a wide range of the anterior eye portion of the eye under examination is photographed is more suitable for positional deviation detection. On the other hand, the captured image in the first photographing unit 115 is required to be an image in which the interference fringes of the tear film are clearly captured by enlarging a part of the eye under examination for diagnosis of dry eye. In order to implement both of the photographing needs based on a common optical axis, the positional relationship between the optical components is adjusted such that, when position adjustment to an appropriate position is made, the first photographing unit 115 has the photographing range obtained by enlarging a tiny part of the anterior eye portion of the eye under examination, and the second photographing unit 125 has, as the photographing range, a wide range of the anterior eye portion of the eye under examination.

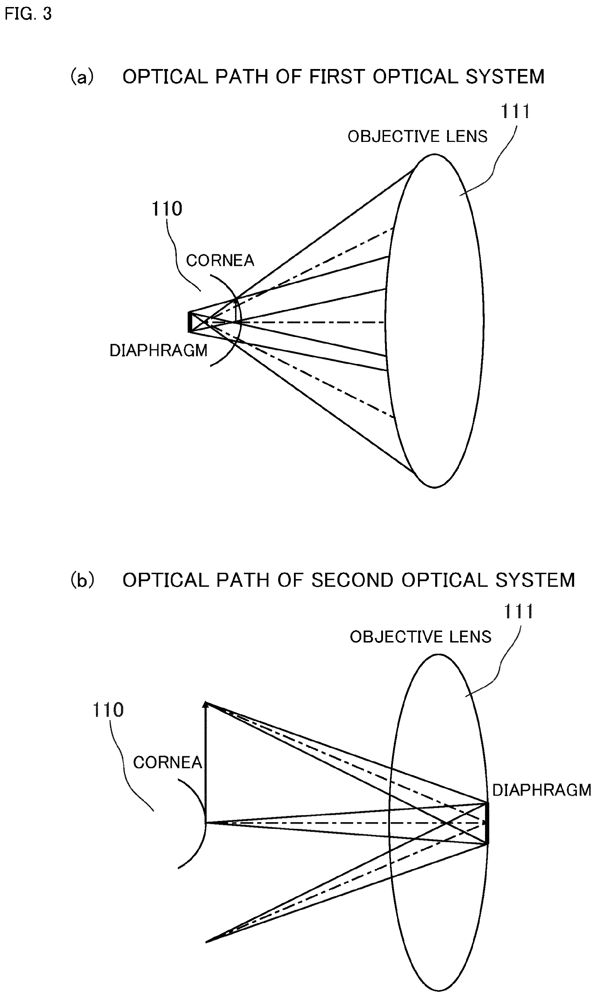

Fig. 3 is an optical path diagram for describing the optical path of the first optical system and the optical path of the second optical system in the tear film photographing device 1 corresponding to the embodiment of the present invention. Specifically, for example, as illustrated in Fig. 3(a), in the first optical system including the first photographing unit 115, an exit pupil is near a corneal curvature center with the intention that a light beam is reflected according to a corneal curvature and a normal direction in order to more easily obtain interference light from a cornea of the eye 110 under examination. On the other hand, as illustrated in Fig. 3(b), the second optical system including the second photographing unit 125 is designed such that an exit pupil is near the objective lens 111 because the objective lens 111 becomes large in a case where the second optical system is implemented with the same exit pupil position as the optical system of the first photographing unit 115 in order to photograph a wider range.

As illustrated in Fig. 2, the Z-direction detection unit 13 includes a second positional deviation detection light source 131 that irradiates the eye under examination with third light, and a light receiving sensor 132 that receives the third light reflected by the anterior eye portion of the eye under examination. The second positional deviation detection light source 131 may be any light source as long as the third light can be reflected by the anterior eye portion of the eye under examination and received by the light receiving sensor 132, and for example, an LED, an organic light emitting diode (OLED), and various lasers are conceivable. The light receiving sensor 132 may be any sensor as long as the light receiving sensor 132 can receive the third light reflected by the anterior eye portion of the eye under examination, and for example, a photodiode (PD) is conceivable. Furthermore, in the present example, the light receiving sensor 132 is preferably a split sensor capable of performing signal conversion while distinguishing between two light receiving regions. As an example, it is conceivable to arrange two photodiodes side by side or adopt a two-segment photodiode. The second positional deviation detection light source 131 is fixed so as to be able to emit the third light at an angle at which an optical axis intersects the center of a pupil of the eye under examination in a case where the anterior eye portion of the eye under examination is present at an ideal position, and the light receiving sensor 132 is fixed such that the third light reflected by the center of the pupil of the eye under examination in a case where the anterior eye portion of the eye under examination is present at the ideal position substantially equally hits the two light receiving regions to obtain equal detection signals. By performing such positioning, the detection signal of one photodiode becomes strong in a case where the eye under examination is at a position farther than the ideal position, and the detection signal of the other photodiode becomes strong in a case where the eye under examination is at a position closer than the ideal position, so that it is possible to detect the positional deviation in the Z direction.

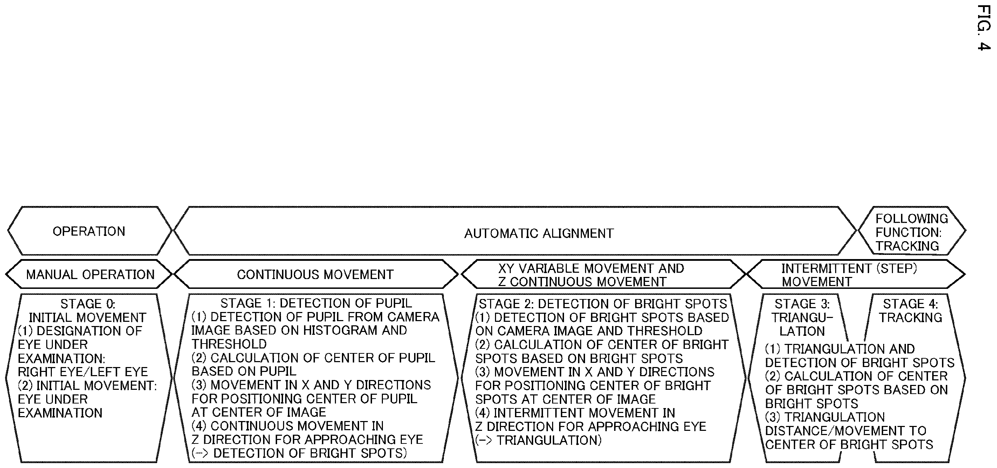

Fig. 4 is an explanatory diagram illustrating an outline of position correction processing in the tear film photographing device 1 corresponding to the embodiment of the present invention. As illustrated in Fig. 4, position correction in the tear film photographing device 1 has three stages of an operation stage by an operator, an automatic alignment stage, and a following/tracking stage. First, in the operation stage by the operator, an initial position is designated based on a manual operation by the operator as a stage 0. Specifically, designation of the eye under examination that is an examination target for determining whether the eye under examination is the right eye or the left eye, and initial movement of the device with respect to the eye under examination are performed.

Next, in the automatic alignment stage, detection of the pupil of the eye under examination for performing coarse position correction in the X and Y directions is performed as a stage 1. First, the position detection image obtained by photographing the anterior eye portion of the eye under examination by the second photographing unit 125 of the second optical system 12 is acquired. Then, the position of the pupil appearing in the acquired position detection image is detected based on image processing. Any means may be used as long as the position of the pupil can be detected. As an example of the image processing for pupil detection, a histogram for luminance values of the position detection image is generated, and binarization processing is performed on the position detection image by counting the number of pixels from a low luminance side and using a luminance value corresponding to a predetermined ratio of the number of pixels to the total number of pixels, for example, a luminance value when reaching 20% of the number of pixels as a threshold. In this case, there is a high possibility of obtaining a binarized image in which a portion of the pupil is processed to be black (luminance 0) and a portion around the pupil is processed to be white (maximum luminance). The position of the pupil is detected based on the binarized image, and then the center of the pupil is calculated. A positional deviation amount between the center of the pupil and the center of the current captured image is detected. Then, a position correction amount necessary for overlapping the center of the captured image with the center of the pupil is calculated. The position correction in the X and Y directions is performed by driving and controlling the X-direction drive mechanism 31 and the Y-direction drive mechanism 32 of the drive mechanism unit 30 based on the calculated position correction amount. The position correction in the X and Y directions is continued until a positional deviation amount between a position of the center of the pupil and a position of the center of the captured image becomes a predetermined target value or less.

When the positional deviation amounts in the X and Y directions reach the predetermined target value or less, coarse position correction in the Z direction is further performed as the stage 1. Specifically, movement control in the Z direction is performed to approach the eye until the light receiving sensor 132 in the Z-direction detection unit 13 detects the signal. Alternatively, bright spots of a Purkinje image generated on the cornea by the second light from the first positional deviation detection light sources 121a to 121c are detected, and the movement control in the Z direction is performed to approach the eye so as to be within a certain range obtained by calculating a distance of Z based on a distance between the bright spots. In order to further increase accuracy, a necessary distance between the bright spots may be corrected by inputting the corneal curvature using another device. Alternatively, control is performed using these two methods in combination.

Next, precise position correction in the X and Y directions is performed as a stage 2 of the automatic alignment stage. The bright spots of the Purkinje image generated on the cornea by the second light from the first positional deviation detection light sources 121a to 121c are detected, and bright spot center coordinates are calculated based on three or two bright spots. Since the center of three bright spot coordinates is equivalent to the center of a circle inscribed in the three bright spots, the bright spot center coordinates can be mathematically obtained based on a relationship between each bright spot and the center. When the bright spot center coordinates are determined, a positional deviation amount from position coordinates of the center of the captured image can be calculated. The position correction amount is calculated based on the calculated positional deviation amount. The position correction in the X and Y directions is performed by driving and controlling the X-direction drive mechanism 31 and the Y-direction drive mechanism 32 of the drive mechanism unit 30 based on the calculated position correction amount. The precise position correction in the X and Y directions is continued until the positional deviation amounts between the bright spot center coordinates and the position coordinates of the center of the captured image become equal to or less than a predetermined target value A.

In a case where the positional deviation amounts between the bright spot center coordinates and the position coordinates of the center of the captured image are equal to or less than the predetermined target value A, a target value B of the positional deviation amount that is smaller than the target value A is set in order to perform more precise control, and a more precise position correction amount is calculated such that the positional deviation amounts become equal to or less than the target value B. At this time, the position correction is performed with higher accuracy than that in the case of performing the position correction toward the target value A, for example, by finely changing a movement amount or shortening a time interval for control. The precise position correction in the X and Y directions is continued until the positional deviation amounts between the bright spot center coordinates and the position coordinates of the center of the captured image become equal to or less than the predetermined target value B.

In a case where the positional deviation amounts in the X and Y directions are equal to or less than the predetermined target value B, next, precise position correction in the Z direction is performed as the stage 2 of the automatic alignment stage. In the precise position correction in the Z direction, a positional deviation amount in the Z direction is calculated based on the detection signals of the two regions of the light receiving sensor 132 that receives the third light from the second positional deviation detection light source 131 in the Z-direction detection unit 13. Specifically, since the light receiving sensor 132 is set such that the third light reflected by the center of the pupil of the eye under examination substantially equally hits the two light receiving regions and the equal detection signals are obtained to position the anterior eye portion of the eye under examination at the ideal position, the position correction amount in the Z direction is calculated based on a difference between the detection signals in the two light receiving regions, and the Z-direction drive mechanism 33 is driven and controlled to perform the position correction in the Z direction. At this time, the position correction in the Z direction is performed based on a finer movement amount than that in the case of the coarse position correction in the stage 1, such as a finer control amount of a drive motor. When the positional deviation amount in the Z direction becomes equal to or less than a predetermined target value, the target in the stage 2, which is the automatic alignment stage with respect to the eye under examination, is reached.

Next, the following/tracking stage is a stage for maintaining the ideal position of the eye under examination reached by the automatic alignment. As a stage 3 and a stage 4, the precise position correction in the X and Y directions up to the stage 2 and the precise position correction in the Z direction are continuously performed. At this time, following/tracking may be implemented by exactly the same control up to the stage 2, or more detailed control than the stage 2 may be performed, such as increasing a monitoring frame rate for operation or setting a drive amount of an actuator to be operated more finely.

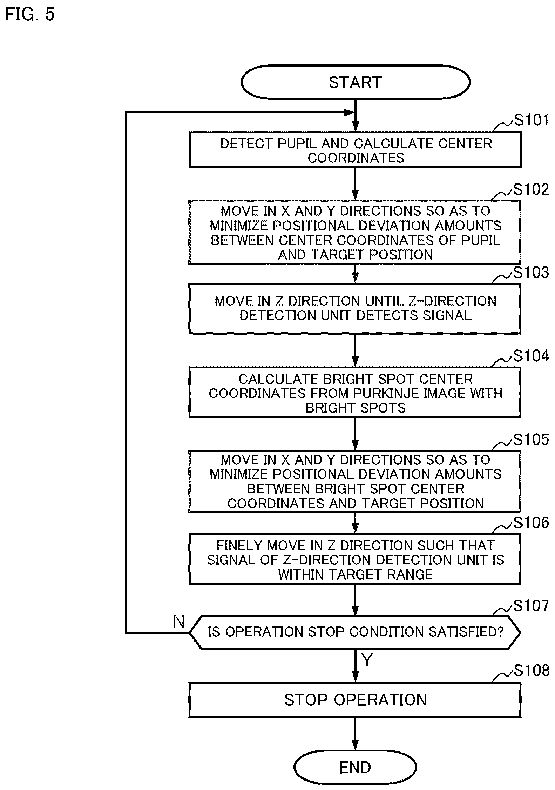

Fig. 5 is a flowchart illustrating a flow of the position correction processing in the tear film photographing device 1 corresponding to the embodiment of the present invention. As illustrated in Fig. 5, in the tear film photographing device 1, the position correction processing is started by detecting the pupil from the eye under examination appearing in the acquired position detection image and calculating center coordinates of the pupil (step S101). Next, the tear film photographing device 1 calculates the positional deviation amounts based on the center coordinates of the pupil and target position coordinates and moves in the X and Y directions (the coarse position correction in the X and Y directions) so as to minimize the positional deviation amounts (step S102). Next, the tear film photographing device 1 moves in the Z direction (the coarse position correction in the Z direction) until the Z-direction detection unit 13 detects the signal (step S103). Next, the tear film photographing device 1 calculates the bright spot center coordinates from the Purkinje image with the bright spots (step S104). Next, the tear film photographing device 1 moves in the X and Y directions (the precise position correction in the X and Y directions) so as to minimize positional deviation amounts between the bright spot center coordinates and a target position (step S105). Next, the tear film photographing device 1 finely moves in the Z direction (the precise position correction in the Z direction) such that the signal of the Z-direction detection unit is within a target range (step S106). Then, the tear film photographing device 1 determines whether or not an operation stop condition is satisfied (step S107). In a case where the operation stop condition is not satisfied (S107-N), the tear film photographing device 1 repeatedly executes steps S101 to S106 to perform following/tracking. In a case where the operation stop condition is satisfied (S107-Y), the tear film photographing device 1 stops the operation (step S108) and then ends the position correction processing.

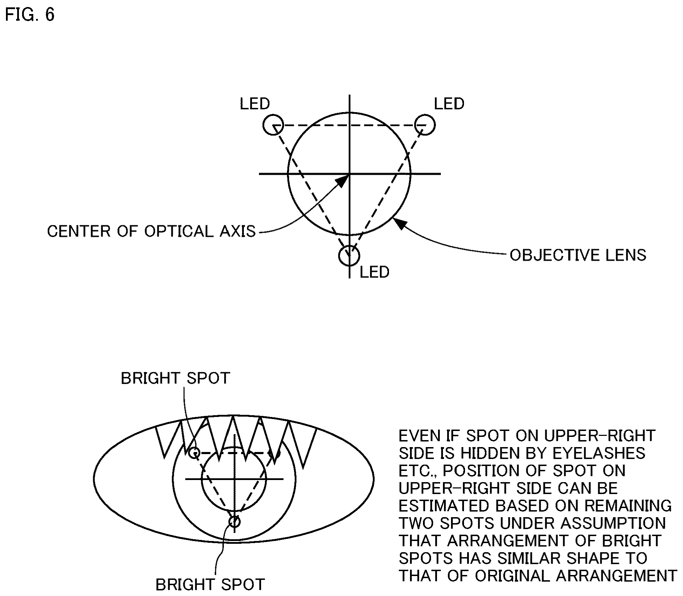

Fig. 6 is an explanatory diagram illustrating an example of installation of the second illumination unit 121 in the tear film photographing device 1 corresponding to the embodiment of the present invention and a method of using the bright spot. In Fig. 6, the first positional deviation detection light sources 121a to 121c as the second illumination unit 121 are three LEDs. As the arrangement of the first positional deviation detection light sources 121a to 121c, the first positional deviation detection light sources 121a to 121c are arranged in an inverted isosceles triangle (inverted equilateral triangle) shape with respect to an outer peripheral portion of the objective lens 111. In a case where the first positional deviation detection light sources 121a to 121c are arranged such that a base of the triangle is at the bottom, there is a possibility that the first positional deviation detection light sources 121a to 121c collide with the nose of the subject when the objective lens 111 faces the eye under examination. Therefore, the inverted triangle shape makes it difficult for the first positional deviation detection light sources to collide with the nose of the subject. In addition, by arranging the first positional deviation detection light sources in the isosceles triangle shape, even if projection of one of three spots on the cornea is blocked by an eyelid or eyelashes of the subject and the Purkinje image cannot be obtained, a position of the hidden one spot can be predicted based on the remaining two spots on the basis of positions of the LEDs arranged around the objective, and as a result, a center position of the Purkinje image can be calculated. Although each bright spot is generated by reflection at the cornea, it can be assumed that the bright spot and the center of the optical axis maintain a similar positional relationship (similar shape) as long as the corneal curvature is not locally distorted with respect to the center of the optical axis. Therefore, even if one of the three spots disappears, it is possible to estimate the position of the remaining third spot and calculate the center of the optical axis at the same time since it is possible to know which one of the two spots each of the bright spots is from a positional relationship of the remaining two spots.

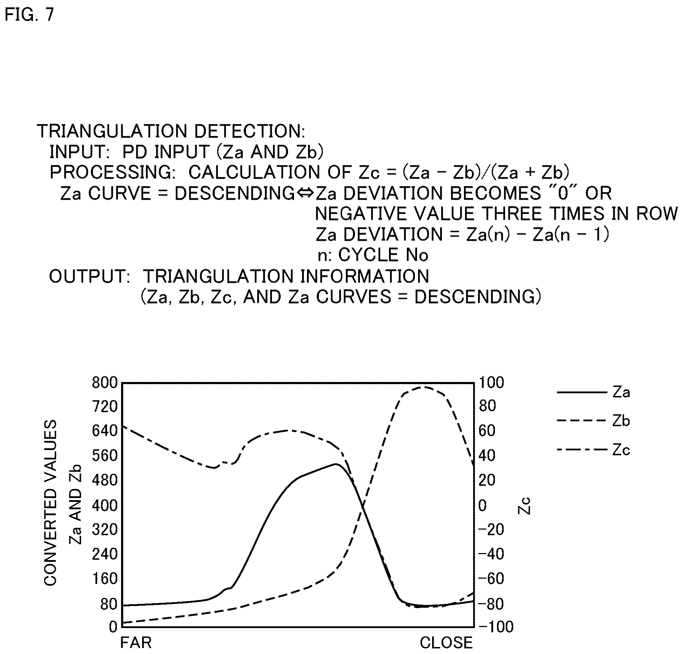

Fig. 7 is an explanatory diagram illustrating a method of calculating the positional deviation amount in the Z direction using the signal from the Z-direction detection unit 13 in the tear film photographing device 1 corresponding to the embodiment of the present invention. First, in a case where the two-segment photodiode is adopted as the light receiving sensor 132 of the Z-direction detection unit 13, two detection signals Za and Zb are obtained. In the calculation of the positional deviation amount in the Z direction, the detection signals Za and Zb are input, and Zc = (Za - Zb)/(Za + Zb) is calculated. In addition, Za deviation = Za(n) - Za(n - 1) is calculated, and it is determined whether or not the Za deviation becomes "0" or a negative value three times in a row. In a case where the Za deviation becomes "0" or a negative value three times in a row, it is determined that a Za curve is descending. In this way, information indicating that Za, Zb, Zc, and Za curves = descending is obtained as the triangulation information. The positional deviation amount in the Z direction is calculated based on the obtained triangulation information. As for the detection signal of the two-segment photodiode, as illustrated in the graph of Fig. 7, an output value of the detection signal of Za first increases as the eye under examination gradually approaches from a far position, Za peaks out at a certain stage and the Za curve descends, and an output value of the detection signal of Zb increases at the same timing. Then, it can be determined that the positional deviation amount in the Z direction is the smallest at a timing at which Za = Zb and Zc is substantially 0.

As described above, the tear film photographing device of the present invention is a tear film photographing device that photographs interference fringes formed on a tear film of an eye under examination, the tear film photographing device being set to include: a first optical system that includes an objective lens that is disposed so as to face the eye under examination, a first illumination unit that irradiates the eye under examination with first light through the objective lens, and a first photographing unit that receives the first light reflected by the tear film of the eye under examination and passing through the objective lens and photographs the interference fringes formed on the tear film; a second optical system that includes a second illumination unit that irradiates the eye under examination with second light, a second light reflection unit that is disposed on an optical path of the first optical system and selectively reflects the second light reflected by a corneal surface of the eye under examination and passing through the objective lens, and a second photographing unit that receives the second light through the second light reflection unit and photographs an anterior eye portion of the eye under examination, the second optical system adjusting a positional relationship between respective optical components such that a photographing range in the second photographing unit is wider than a photographing range in the first photographing unit; and a positional deviation amount calculation unit that calculates a positional deviation amount from a proper position of the first optical system with respect to the eye under examination based on an image of the anterior eye portion obtained by the second optical system. Therefore, it is possible to provide the tear film photographing device capable of implementing a position detection function for the eye under examination and an automatic alignment function without an increase in size of the entire device. In addition, when the automatic alignment function is implemented based on a position detection image obtained by photographing the wider range in a state in which the positional relationship between the respective optical components is adjusted such that the photographing range in the second photographing unit is wider than the photographing range in the first photographing unit, alignment for the first photographing unit that photographs the narrower range is also completed at a time point when the position correction is completed, so that it is possible to immediately shift to the photographing of the interference fringes of the eye under examination.

In addition, the second illumination unit includes a plurality of first positional deviation detection light sources that are disposed around the objective lens and emit the second light, the second optical system captures the image of the anterior eye portion and photographs a plurality of bright spots of a Purkinje image of the second light, and the positional deviation amount calculation unit is set to calculate the positional deviation amounts in the X direction and the Y direction based on the image of the anterior eye portion and the plurality of bright spots. Therefore, it is possible to provide the tear film photographing device capable of implementing the position detection function for the eye under examination and the automatic alignment function without an increase in size of the entire device.

In addition, since the positional deviation amount calculation unit is set to have a first positional deviation amount calculation function of detecting the center of a pupil of the eye under examination and calculating the positional deviation amounts in the X direction and the Y direction based on the image of the anterior eye portion obtained by the second optical system, it is possible to shorten a time until the target value is reached by position correction by first performing coarse position correction in the X and Y directions based on information regarding a position of the pupil.

In addition, since the positional deviation amount calculation unit is set to have a second positional deviation amount calculation function of detecting the center of the plurality of bright spots and calculating the positional deviation amounts in the X direction and the Y direction based on images of the plurality of bright spots obtained by the second optical system, it is possible to perform the precise position correction in the X and Y directions.

In addition, since the positional deviation amount calculation unit is set to have a third positional deviation amount calculation function of calculating a distance between the images of the respective bright spots among the images of the plurality of bright spots obtained by the second optical system and calculating the positional deviation amount in the Z direction based on the distance, it is possible to shorten the time until the target value is reached by the position correction by first performing the coarse position correction in the Z direction.

In addition, the tear film photographing device includes a Z-direction detection unit including a second positional deviation detection light source that is disposed around the objective lens and irradiates the eye under examination with third light and a light receiving sensor that is disposed around the objective lens and receives the third light reflected by the anterior eye portion of the eye under examination, and the positional deviation amount calculation unit is set to have a fourth positional deviation amount calculation function of detecting the positional deviation amount in the Z direction based on a light receiving position for the third light in the light receiving sensor. Therefore, it is possible to perform the precise position correction in the Z direction based on the principle of triangulation.

In addition, since the plurality of first positional deviation detection light sources are set to include three first positional deviation detection light sources disposed at positions of respective vertexes of an inverted isosceles triangle shape when viewed from a front side of the objective lens, it is possible to reduce a risk that the first positional deviation detection light sources come into contact with the nose of the subject, and as the first positional deviation detection light sources are arranged in the inverted isosceles triangle shape, it is possible to calculate the center coordinates of the bright spots after estimating the position of the bright spot that does not appear even in a situation where one of the bright spots does not appear.

### Reference Signs List

- 1: Tear film photographing device
- 10: Optical system unit
- 11: First optical system
- 110: Eye under examination
- 111: Objective lens
- 112: First illumination unit
- 113: Half mirror
- 114: Imaging lens
- 115: First photographing unit
- 12: Second optical system
- 121: Second illumination unit
- 121a to 121c: First positional deviation detection light source
- 122: Second light reflection unit
- 123: Mirror
- 124: Imaging lens
- 125: Second photographing unit
- 13: Z-direction detection unit
- 131: Second positional deviation detection light source
- 132: Light receiving sensor
- 20: Control unit
- 21: Image information acquisition unit
- 22: Storage unit
- 23: Positional deviation amount calculation unit
- 24: Position correction unit
- 30: Drive mechanism unit
- 31: X-direction drive mechanism
- 32: Y-direction drive mechanism
- 33: Z-direction drive mechanism
- 40: Monitor

## Claims

1. A tear film photographing device that photographs interference fringes formed on a tear film of an eye under examination, the tear film photographing device comprising:
a first optical system that includes an objective lens that is disposed so as to face the eye under examination, a first illumination unit that irradiates the eye under examination with first light through the objective lens, and a first photographing unit that receives the first light reflected by the tear film of the eye under examination and passing through the objective lens and photographs the interference fringes formed on the tear film;
a second optical system that includes a second illumination unit that irradiates the eye under examination with second light, a second light reflection unit that is disposed on an optical path of the first optical system and selectively reflects the second light reflected by a corneal surface of the eye under examination and passing through the objective lens, and a second photographing unit that receives the second light through the second light reflection unit and photographs an anterior eye portion of the eye under examination, the second optical system adjusting a positional relationship between respective optical components such that a photographing range in the second photographing unit is wider than a photographing range in the first photographing unit; and
a positional deviation amount calculation unit that calculates a positional deviation amount from a proper position of the first optical system with respect to the eye under examination based on an image of the anterior eye portion obtained by the second optical system.

2. The tear film photographing device according to claim 1, wherein
the second illumination unit includes a plurality of first positional deviation detection light sources that are disposed around the objective lens and emit the second light,
the second optical system captures the image of the anterior eye portion and photographs a plurality of bright spots of a Purkinje image of the second light, and
the positional deviation amount calculation unit calculates the positional deviation amounts in an X direction and a Y direction based on the image of the anterior eye portion and the plurality of bright spots.

3. The tear film photographing device according to claim 2, wherein the positional deviation amount calculation unit has a first positional deviation amount calculation function of detecting a center of a pupil of the eye under examination and calculating the positional deviation amounts in the X direction and the Y direction based on the image of the anterior eye portion obtained by the second optical system.

4. The tear film photographing device according to claim 2 or 3, wherein the positional deviation amount calculation unit has a second positional deviation amount calculation function of detecting a center of the plurality of bright spots and calculating the positional deviation amounts in the X direction and the Y direction based on images of the plurality of bright spots obtained by the second optical system.

5. The tear film photographing device according to claim 2 or 3, wherein the positional deviation amount calculation unit has a third positional deviation amount calculation function of calculating a distance between the images of the respective bright spots among the images of the plurality of bright spots obtained by the second optical system and calculating the positional deviation amount in a Z direction based on the distance.

6. The tear film photographing device according to claim 2 or 3, wherein
the tear film photographing device comprises a Z-direction detection unit that includes a second positional deviation detection light source that is disposed around the objective lens and irradiates the eye under examination with third light and a light receiving sensor that is disposed around the objective lens and receives the third light reflected by the anterior eye portion of the eye under examination, and
the positional deviation amount calculation unit has a fourth positional deviation amount calculation function of detecting the positional deviation amount in a Z direction based on a light receiving position for the third light in the light receiving sensor.

7. The tear film photographing device according to claim 2 or 3, wherein the plurality of first positional deviation detection light sources include three first positional deviation detection light sources disposed at positions of respective vertexes of an inverted isosceles triangle shape when viewed from a front side of the objective lens.
